# EUROPEAN PATENT SPECIFICATION

(11) **EP 3 645 505 B1**
(45) Date of publication and mention of the grant of the patent: **29.05.2024**
(21) Application number: 18825377.7
(22) Date of filing: 22.06.2018
(51) Int. Cl.: C07C 227/40, C07C 209/86, C07C 51/48, C07C 51/47, C07C 51/43, B01D 3/36, B01D 11/00, B01D 11/04, B01D 3/00, C07C 211/07, C07C 227/42, C07C 51/42, C07C 55/18, C07C 53/126, C07C 229/08, C07C 55/21, C07C 51/46, B01D 3/10

(54) **PROCESS FOR THE SEPARATION OF LONG CHAIN AMINO ACIDS AND DIBASIC ACIDS**
VERFAHREN ZUR TRENNUNG VON LANGKETTIGEN AMINOSÄUREN UND DIBASISCHEN SÄUREN
PROCÉDÉ DE SÉPARATION D'ACIDES AMINÉS À CHAÎNE LONGUE ET D'ACIDES DIBASIQUES À CHAÎNE LONGUE

(30) Priority: 28.06.2017 US 201715635874; 07.07.2017 US 201715644665
(43) Date of publication of application: 06.05.2020
(73) Proprietor: Vitaworks IP, LLC, North Brunswick, NJ 08902 (US)
(72) Inventor: HU, Songzhou, North Brunswick, NJ 08902 (US)
(74) Representative: Gill Jennings & Every LLP
(86) International application number: PCT/US2018/039132
(87) International publication number: WO 2019/005630

(56) References cited:
- EP-A1- 3 381 888
- WO-A1-2017/088218
- SU-A1- 1 209 680
- US-A- 2 700 054
- US-A- 4 496 736
- US-A- 5 770 765
- BOWEN C. V.: "Distribution of Anabasine between Certain Organic Solvents and Water", Industrial & Engineering Chemistry, vol. 46, no. 6, 1949, pages 1295-1296, XP055557268,

## Description

### TECHNICAL FIELD

The present invention relates to a process for the separation of long chain amino acids, long chain dibasic acids, alkylamines, and alkanoic acids.

### BACKGROUNDS OF THE INVENTION

Long chain saturated aliphatic amino acids, lactams, and dibasic acids are important monomers for long chain nylons and engineering plastics. Nylons are a class of polymers that contain amide bond on their backbone of chains. Nylons are one of the most widely used, most numerous in types, and most consumed class of engineering plastics.

Because of their unusual molecular structure, long chain nylons possess extraordinary physical properties, i.e., higher mechanical strength than metal, low hygroscopicity, excellent resistance to oil, low temperature, abrasion, and chemical corrosion, and most importantly, easy to fabricate. Long chain nylons are made into many kinds of plastics products, spun to fibers, and stretched to thin films. Long chain nylons are also used in paints and hot melt adhesives. Hence, long chain nylons find wide applications in automobile, electrical, electronic, telecommunications, petrochemical, and aerospace industries.

Long chain amino acids and lactams are used industrially as monomers to produce nylon-9, nylon-11, and nylon-12.

Long chain dibasic acids are condensed with diamines industrially as starting materials to produce nylon-610, nylon-612, nylon-510, nylon-512, nylon-1010, and nylon-1212.

WO 2017/088218 and the co-pending U.S.S.N. 15/601,556 both by the present inventor, disclose a novel process for the coproduction of long chain amino acids and dibasic acids from keto fatty acid derivatives. According to the disclosed process, long chain keto fatty acid derivatives are reacted with hydroxylamine to form an oxime derivative, which is subjected to the Beckmann rearrangement to yield a mixture of two amide derivatives. These amide derivatives are hydrolyzed to a mixture of products containing long chain amino acids and dibasic acids, which are isolated by a process of step-wise neutralization in a highly dilute concentration. Thus, a substantial amount of energy is required for the concentration, so that the process is not economical.

Moreover, the present inventor has found that long chain amino acids and dibasic acids of required quality for the production of polyamides cannot be obtained, if the process according these prior disclosures is applied for commercial starting materials, which contain various amount of other fatty acids. Apparently, these impurities contaminate intended products and thus demand a process for their removal from final products of required purity.

Hydrolysis of the mixed amide derivatives from the Beckmann rearrangement yields not only long chain amino acids and dibasic acids, but also short chain alkyl amines and alkanoic acids. There is a lack of any method for the separation and recovery of these short chain products from the mixture of the hydrolysis reaction.

It is desirable to have a process for the separation of each component to their required purity from their complex mixture to achieve an economical process and to reduce or eliminate the disposal of waste stream.

It is an object of the present invention to disclose a process for the separation of long chain amino acids, long chain dibasic acids, alkyl amines, and alkanoic acids, and for the recovery of other fatty acids present in the commercial starting materials, such as stearic acid, and impurities generated as byproducts of the production reactions. By the process of the present invention, long chain amino acids and long chain dibasic acids are separated simply, efficiently, and economically with high yields and excellent purity.

It is another object of the present invention to disclose a process for the recovery of long chain amino acids and inorganic salts from aqueous waste mother liquor. As a result, there is no aqueous waste discharge from production process.

### DESCRIPTION OF THE DRAWINGS

FIG. **1****.** Schematic flowchart for the separation of long chain amino acid, dibasic acid, alkylamine, and alkanoic acid from their mixture in the case of an alkali hydroxide hydrolysis.

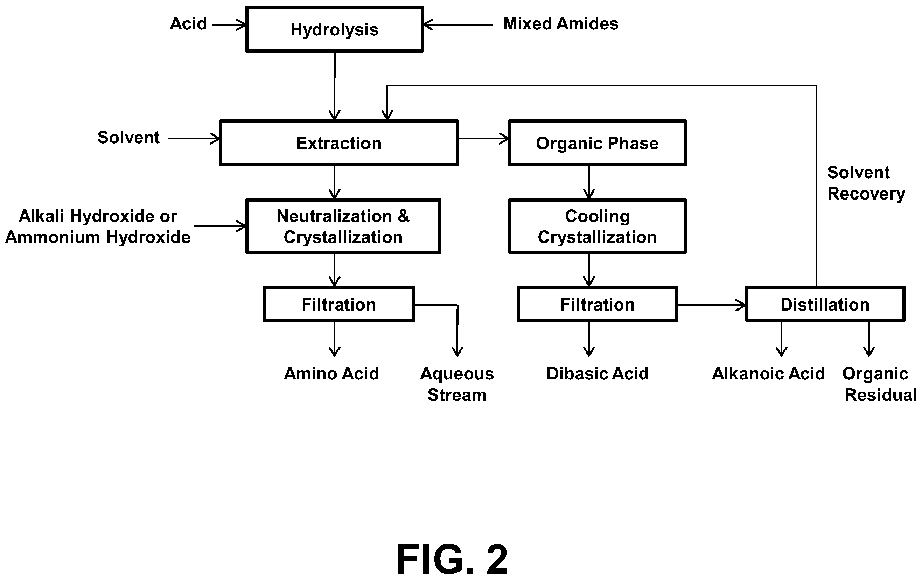

FIG. **2****.** Schematic flowchart for the separation of long chain amino acid, dibasic acid, alkylamine, and alkanoic acid from their mixture in the case of an acid hydrolysis.
FIG. **3****.** Schematic flowchart for the recovery of long chain amino acid and alkali salt from waste aqueous stream with the aid of an alkali hydroxide.
FIG. **4****.** Schematic flowchart for the recovery of long chain amino acid and alkali salt from waste aqueous stream with the aid of an acid.
FIG. **5****.** Schematic flowchart for the treatment of aqueous stream to recover alkylamine, long chain amino acid, and alkali salt for an acid hydrolysis of mixed amide derivatives.
FIG. **6****.** Solubility curve of 11-aminoundecanoic acid in water at neutral pH, 1 M solution of sulfuric acid, and 2 M solution of sodium hydroxide.
FIG. **7****.** Solubility curve of heptanoic acid in a 6 M solution of sodium hydroxide and dodecanedioic acid in a 3 M solution of sodium hydroxide.
FIG. **8****.** Solubility of 11-aminoundecanoic acid and dodecanedioic acid in aqueous ethanol solution of 2 M sodium hydroxide at different concentration of ethanol.

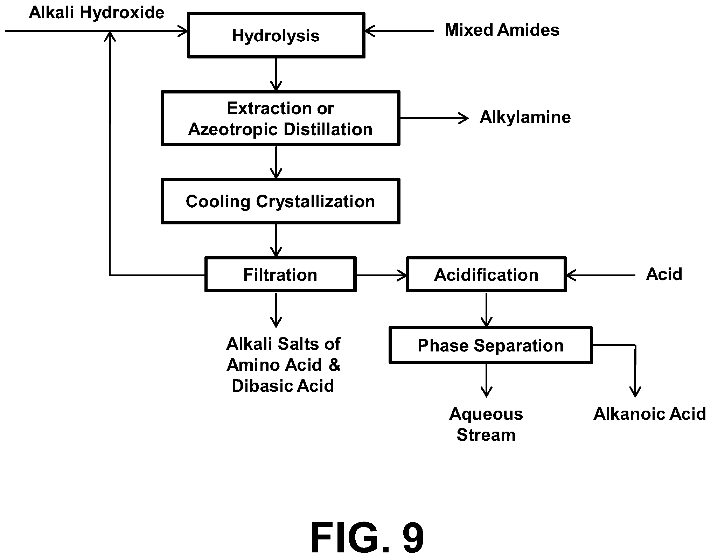

FIG. **9****.** Schematic flowchart for the separation of alkali salts for long chain amino acid and dibasic acid from a mixture of an alkali hydroxide hydrolysis.

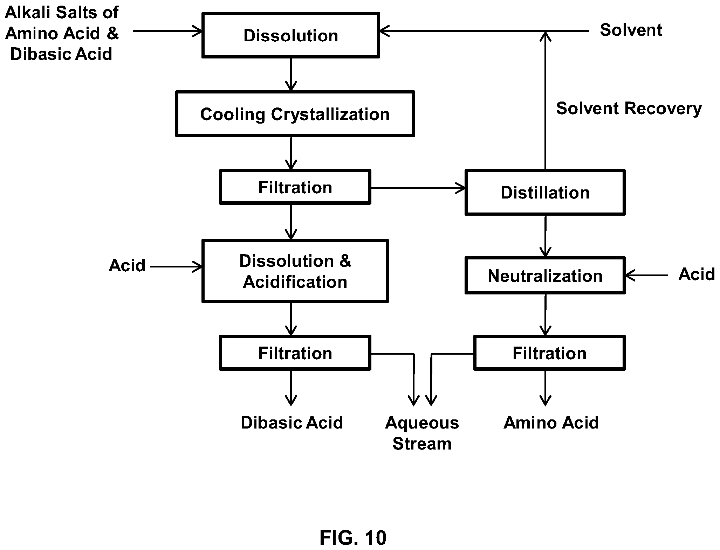

FIG. **10****.** Schematic flowchart for the separation of alkali salt of long chain amino acid and long chain dibasic acid from their mixture by use of aqueous solvent.
FIG. **11****.** Schematic flowchart for the separation of long chain amino acid and dibasic acid from a mixture of an alkali hydroxide hydrolysis in an aqueous solvent.
FIG. **12****.** Schematic flowchart for the separation of inorganic alkali salt and the recovery of the alkali salts of long chain amino acids and dodecanedioic acid from waste aqueous stream.

### DESCRIPTION OF THE INVENTION

The invention is defined in the appended claims.

Hydrolysis of the mixed amide derivatives of the following structures: 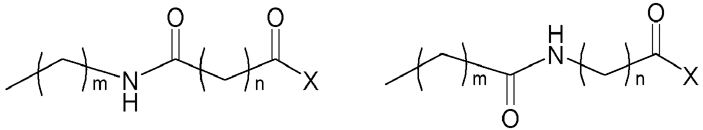 from the Beckmann rearrangement of oxime fatty acid derivatives can be carried out with either an acid or a base to yield a mixture of main products of the following structures: 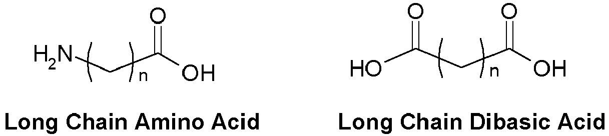 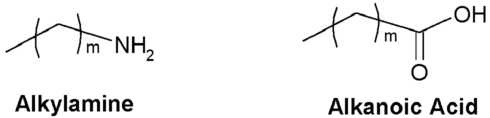 wherein m is an integral from 0 to 10, n is an integral from 6 to 20; X is OR or NR₁R₂, wherein OR is OH, Ci-Cs monohydric alcohol or Ci-Cs polyhydric alcohol, and R₁ and R₂ are each independently hydrogen or Ci-Cs alkyl group.

When m = 5, n = 10, the main products are 11-aminoundecanoic acid, dodecanedioic acid, hexylamine, and heptanoic acid. Because the starting material of commercial grade is obtained from castor oil, significant amount of stearic acid is also present as an impurity in the mixture of products.

When m = 7, n = 8, the main products are 9-aminononanoic acid, sebacic acid, octylamine, and pelargonic acid.

When m = 5, n = 12, the main products are 13-aminotridecanoic acid, tetradecanedioic acid (brassylic acid), hexylamine, and heptanoic acid.

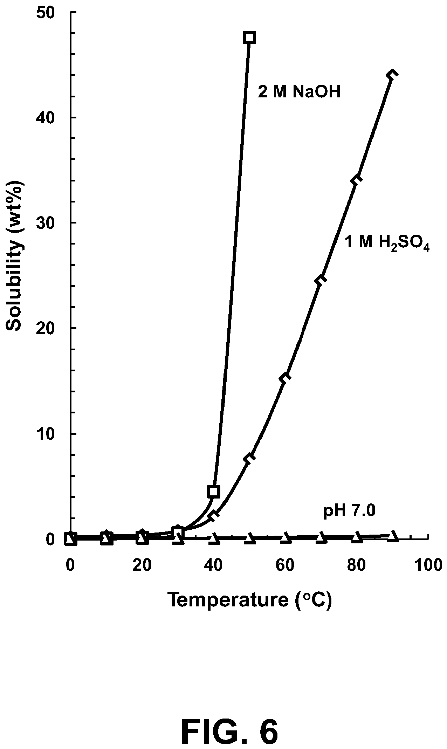

When the hydrolysis reaction of mixed amide derivatives from the Beckmann rearrangement is performed in the presence of alkali hydroxide, main products other than alkylamine are obtained in the form of their alkali salts. It was observed that a starting suspension of the mixed amide derivatives in a solution of alkali hydroxide is changed to a clear solution at a temperature of 60 °C or above after the hydrolysis. Upon cooling, the clear solution becomes a pasty, non-stirrable cake, because alkali salts of long chain amino acids and dibasic acids are nearly insoluble in an alkali solution as shown in Fig. 6.

The alkali metals are lithium, sodium, potassium, or cesium.

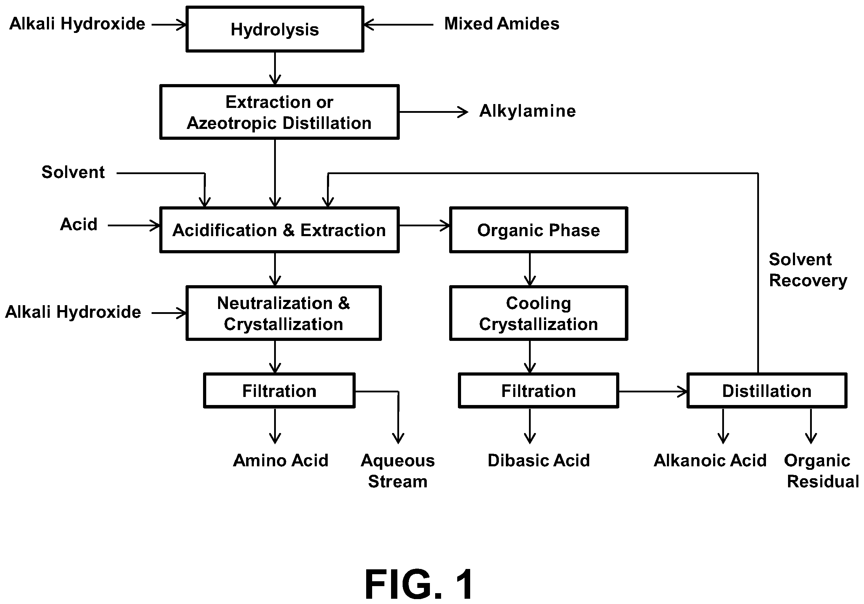

The process according to the present invention, illustrated in FIG. 1 for the separation of each component in a mixture of the hydrolysis reaction by the method of alkali hydroxide, starts with removal of low-boiling components and alkylamine.

The low boiling component comes from alcohols, i.e., methanol or ethanol, commonly used in the starting material of keto fatty acid esters. If the mixed amide derivatives are carboxylic acid, little or no low boiling component is present in the mixture.

These low boiling alcohols, formed by the hydrolysis of esters, are distilled off from the reaction mixture. Distillation of these low boiling alcohols can be carried out under normal pressure, increased pressure, or reduced pressure, during or after the hydrolysis reaction.

Some alkylamines, in particular, of C₁ to C₅, are of lower boiling point, and they are distilled off along with alcohols. These alkylamines can be separated from alcohols according to methods known in prior art.

For the production of 11-aminoundecanoic acid and dodecanedioic acid, hexylamine is one of the main products. Hexylamine is found to form an azeotrope with water and can be separated from the solution by azeotropic distillation. Upon cooling, the distillate separates into an upper phase of nearly pure hexylamine and an aqueous phase containing not more than 2% of hexylamine. Hexylamine can also be separated from the mixture by steam distillation or steam stripping. Complete separation is accomplished when the distillate at the overhead becomes nearly neutral at a pH of 7-8.

The hexylamine distillate contains a small percentage of water and can be dried with a drying agent, and preferably, by azeotropic distillation of a small amount of hexylamine to remove the water in hexylamine.

Hexylamine and alkylamines of more than C₇ can also be separated from the hydrolysis solution by extraction with an extractant solvent. These alkylamines show excellent partition properties between an organic extractant phase and the strongly alkaline aqueous mixture of the hydrolysis reaction. Suitable extractant solvents are selected from the classes of ester, aliphatics, aromatics, ethers, ketones, and water-insoluble amines. Preferably, selected extractant solvent is the same as the solvent chosen for the next stage of the process according to the present invention.

Long chain amino acids and dibasic acids exhibit similarly low solubility through a wide range of pH from 2 to 10 at room temperature. Their separation from each other necessitates a high dilution with great difficulty even when their mixture is not contaminated by other impurities of similar properties, such as fatty acids. When the commercial starting materials, which invariably contain many other fatty acids, are used in the process according to previous disclosure, the products, long chain amino acids and dibasic acids, are always contaminated with these fatty acids.

The present inventor carried out extensive studies to overcome the inherent problems imposed by their little difference in solubility for long chain amino acids and dibasic acids and found in the present invention that the solubility of long chain amino acids can be greatly increased by reacting these amino acids with an acid to form an acid salt at increased temperature. At the same time, alkali salts of long chain dibasic acids and fatty acids in the solution are turned into their free carboxylic acids, which can be dissolved in an organic solvent. Complete separation of these long chain amino acids from long chain dibasic acids and fatty acids is thus accomplished by forming an aqueous solution of an acidic salt of these long chain amino acids and an organic extractant phase rich in long chain dibasic acids, short chain alkanoic acids, and fatty acids.

Suitable acids are an acid of a pKa <5.0. These acids areinorganic acids, i.e., hydrochloric acid, hydrobromic acid, hydroiodic acid, sulfuric acid, nitric acid, phosphoric acid; alkyl and aryl sulfonic acids, i.e., methanesulfonic acid, ethanesulfonic acid, propanesulfonic acid, isethionic acid, benzenesulfonic acid, toluenesulfonic acid, xylenesulfonic acid, and sulfamic acid; organic carboxylic acids: malic acid, maleic acid, tartaric acid, glycolic acid, lactic acid, citric acid, oxalic acid, formic acid, acetic acid, and propionic acid. One or a mixture of two or more of these acids can be used to form an acidic salt of long chain amino acids.

Preferably, the acid is selected from one of the inorganic acids, and most preferably, sulfuric acid.

The aim of acidification is to completely convert alkali salts of long chain dibasic acids, short chain alkanoic acids, and fatty acids into free carboxylic acids and to form an acid salt of long chain amino acid, so as to ensure complete dissolution of long chain amino acid in aqueous phase and long chain dibasic acid in an organic extractant phase.

Organic solvents suitable for extracting dibasic acids and fatty acids are water-insoluble and belong to the classes of ester, aliphatics, aromatics, ethers, alcohols of C₄ to C₁₀, and ketones of C₄ to C₁₀. Useful solvents include butyl formate, isobutyl formate, butyl acetate, isobutyl acetate, propyl acetate, isopropyl acetate, ethyl acetate, ethyl propionate, octyl acetate, benzene, toluene, xylene, cumene, anisole, diethyl ether, diisopropyl ether, dibutyl ether, methyl tert-butyl ether, ethyl tert-butyl ether, methyl tetrahydrofuran, petroleum ether, cyclohexane, dichloroethane, methylene chloride, chloroform, carbon tetrachloride, and trifluoromethylbenzene, n-butanol, isobutanol, amyl alcohol, isoamyl alcohol, hexanol, cyclohexanol, 2-ethylhexanol, isooctanol, sec-octanol, butanone, pentanone, hexanone, cyclohexanone, methyl isobutyl ketone. A single solvent or a mixture of two or more solvents can be used as extractant solvent.

Selected extractant solvent is expected to have good solubility of long chain dibasic acid and fatty acid at higher temperature, low or little solubility at lower temperature for the long chain dibasic acid and good solubility for fatty acid at lower temperature to ensure an effective separation of long chain dibasic acid from other fatty acids rich in the organic phase.

Preferably, the extractant solvent is toluene.

The amount of extractant solvent is not limited, but is greater than the effective amount for the dissolution of dibasic acids and fatty acid impurities.

Temperature to perform acidification and extraction is in the range from 50 °C to the boiling point of the mixture of extractant organic phase rich in long chain dibasic acid and fatty acid and below 100 °C under normal pressure. Acidification and extraction can also be carried out at elevated temperature under pressure, but pressure equipment will be needed for the process.

Preferably, acidification and extraction are performed at a temperature from 60 °C to 95 °C, and most preferably at a temperature from 80 °C to 90 °C. At higher temperature, the higher solubility of long chain dibasic acid in the extractant solvent is advantageous in reducing the amount of the extractant solvent used.

There is no preference as to how an acid and an extractant solvent are introduced into the solution of alkali salts of long chain amino acid and dibasic acid that have been freed of alkylamine. An acid and an extractant solvent can be added concomitantly, sequentially, continuously, semi-continuously, or batch wise.

When the acidification and extraction are performed according to the process of the present invention, good phase separation is achieved. The present inventor unexpectedly found that extractant solvent extracts nearly all colored materials into extractant phase, leaving behind a colorless aqueous solution of the acid salt of long chain amino acid, which provides an added advantage in greatly simplifying the purification of long chain amino acids.

The present inventor found that a middle phase between the upper extractant phase and lower aqueous phase is formed in some cases and contains predominantly the acid salt of long chain amino acid and the acid salt of hexylamine, if hexylamine is not removed or removed incompletely from the hydrolysis solution. This middle phase is formed, especially, when the aqueous solution contains a high concentration of alkali salt. However, the middle phase can be effectively separated and combined with aqueous phase to recover long chain amino acid. Alternatively, after separating the aqueous phase, the middle phase is dissolved with deionized water at increased temperature.

Although the aqueous solution of the acid salt of long chain amino acid is nearly colorless, to further improve the quality of the isolated product, the solution can be treated with activated carbon to decolorize and to absorb minor impurities. The treatment can be carried out from 50 °C to the boiling point for a period from a few minutes to a few hours, preferably 30 minutes to 2 hours, most preferably for 1 hour. After filtration, a clear colorless solution is obtained.

In order to isolate long chain amino acid, the strongly acidic aqueous solution is neutralized with a basic agent to near neutral acidity in a pH range from 5 to 9. More preferably, the pH is in the range of 6 to 8. The neutralization is performed at a temperature from 50 °C to the boiling point of the solution, preferably from 60 to 90 °C, most preferably from 70 °C to 80 °C. Neutralization at this most preferred temperature produces larger crystals that will facilitate solid-liquid separation. After cooling to 30 °C to 40 °C, the product, long chain amino acid, is precipitated and separated by means of solid-liquid separation, i.e., filtration or centrifuge, to yield a mother liquor containing inorganic salt and a small amount of long chain amino acid.

The basic agent is selected from the group consisting of ammonia, alkali and ammonium salts of hydroxide, bicarbonate, carbonate, sulfite, bisulfite, and carboxylate. A single agent or a mixture of two or more agents can be used. Preferably, the basic agent is an alkali hydroxide, and most preferably, the same agent used in the hydrolysis reaction of the mixed amide derivatives.

The most preferable basic agent is sodium hydroxide.

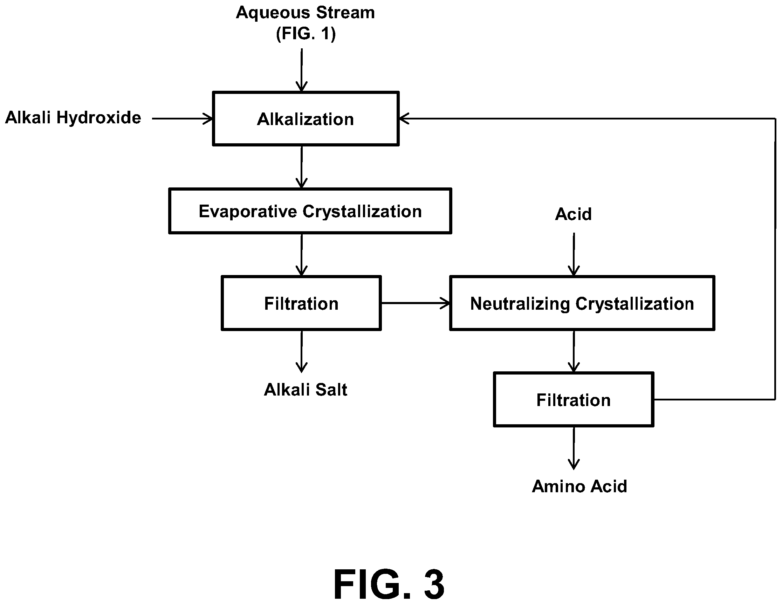

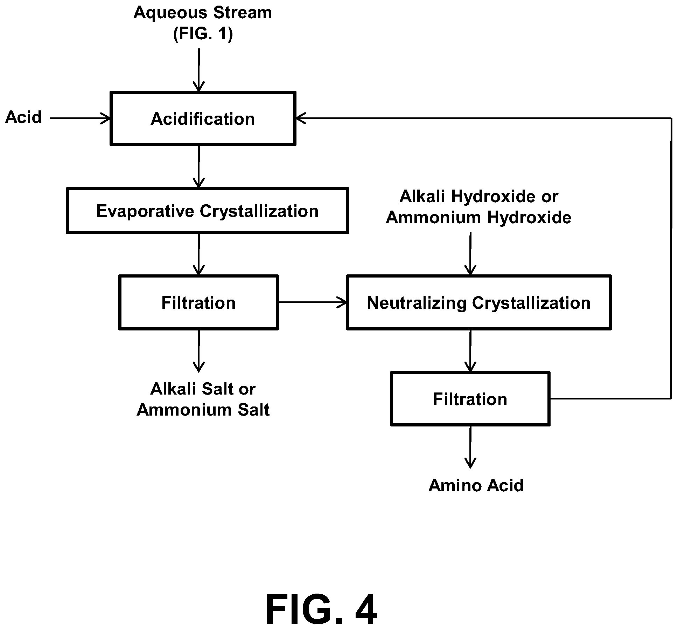

Treatment of the mother liquor after the isolation of long chain amino acid is illustrated in Fig. 3 and Fig. 4 to achieve a complete separation of inorganic salt and full recovery of long chain amino acid with the aid of an alkali hydroxide or an acid, respectively.

Since long chain amino acid, e.g., 11-aminoundecanoic acid, has relatively constant solubility, evaporative concentration of the mother liquor will result in the crystallization of inorganic salt, in particular, sodium sulfate, along with valuable long chain amino acid. To overcome this difficulty, the present inventor found that the solubility of 11-aminoundecanoic acid can be drastically increased by increasing or lowering the pH at increased temperature as illustrated in Fig. 6. In fact, alkali salt of 11-aminoundecanoic acid becomes freely soluble in 2 M solution of sodium hydroxide at about 50 °C. This finding greatly facilitates the separation of inorganic salt, most preferably, sodium sulfate, and the recovery of long chain amino acids.

Although the solubility of long chain amino acid can be increased by both an acid and an alkali hydroxide, it is preferable to use an alkali hydroxide, because alkali salt is non-corrosive to commonly used process equipments made of stainless steel.

After adjusting pH of the aqueous stream with an alkali hydroxide, the mother liquor is concentrated to crystallize inorganic salt, most preferably, sodium sulfate, at a temperature from 40 °C to the boiling point of the solution. Evaporative crystallization can be carried out under normal, reduced, or increased pressure, continuously or in batch. The crystallized salt is removed from the saturated solution by means of solid-liquid separation, e.g., filtration or centrifuge.

The basic mother liquor after removal of alkali salt is neutralized with an acid to a neutral pH. The dissolved long chain amino acid precipitates and can be recovered by means of solid-liquid separation, and the mother liquor is recycled.

The acid used in this step can be selected from the class of inorganic acids, organic carboxylic acids, organic sulfonic acids, sulfamic acid. Preferably, one of the inorganic acids is selected. More preferably, the same acid is used as in previous step. Most preferably, the acid is sulfuric acid.

The extractant phase rich in long chain dibasic acid, short chain alkanoic acid, and fatty acid after the separation of aqueous phase is cooled to a lower temperature in the range of 0 °C to 50 °C, more preferably 0 °C to 30 °C, most preferably 10 °C to 20 °C to crystallize long chain dibasic acid, which can be separated by means of solid-liquid separation. Although the extractant phase and filtration mother liquor is dark in color, the product is nearly white in color and free of any other fatty acids, such as stearic acid.

The mother liquor is distilled to recover extractant solvent and the residual is distilled under vacuum to recover short chain alkanoic acid, e.g., heptanoic acid, in nearly pure form.

In one embodiment of the present invention, the extractant phase is first concentrated by distillation, then cooled to crystallize long chain dibasic acid in an increased yield.

In another embodiment of the present invention, the extractant phase is first distilled to recover extractant solvent, then distilled under vacuum to recover short chain alkanoic acid. To the distillation residual is added an organic solvent to dissolve the residual by heating, then to crystallize long chain dibasic acid by cooling. The solvent is most preferably the original extractant solvent, so that no mixture of different solvents will result to simplify the overall process.

In a further embodiment of the present invention, the extractant phase is distilled to recover solvent and the residual is added to a lower alcohol, in particular methanol or ethanol, most preferably, methanol, in the presence of an esterification catalyst to yield a mixture of methyl esters of alkanoic acid, long chain dibasic acid, and other fatty acid originating from starting materials. These methyl esters are then fractionally distilled to obtain each component in pure form and are freed of any colored materials. These pure methyl esters are marketed directly or can be hydrolyzed to their respective carboxylic acid according to process known in prior art.

Alternatively, the mixture of methyl esters is distilled to a mixture that is freed of any colored materials. The distilled mixture of methyl esters are then hydrolyzed to a mixture of alkanoic acid, long chain dibasic acid, and fatty acid, which can be separated according to the process of the present invention.

In the case of producing 11-aminoundecanoic acid and dodecanedioic acid according to the process of the present invention, the distillation residual is black in color and contains stearic acid from the starting material of castor oil and a small amount of dodecanedioic acid. This dark residual is reacted with a lower alcohol, most preferably, methanol, in the presence of an acid catalyst to form methyl esters. The mixed methyl esters are fractionally distilled to yield colorless methyl esters of stearic acid and dodecanedioic acid. The recovered methyl stearate is either hydrolyzed to stearic acid or marketed as a commercial product, while the methyl ester of dodecanedioic acid is hydrolyzed to obtain dodecanedioic acid.

When the hydrolysis reaction of mixed amide derivatives from the Beckmann rearrangement is performed with an acid, most preferably, sulfuric acid, alkyl amine and long chain amino acid are obtained in the form of their acid salts, while long chain dibasic acid and fatty acid exist in the form of free carboxylic acid.

After the hydrolysis reaction proceeds to completion, water and an extractant solvent are introduced into the suspension to dissolve the acid salts of long amino acid and alkylamine and to transfer the long chain dibasic acid, short chain alkanoic acid, and other fatty acid into an extractant phase.

There is no preference as to how water and extractant solvent are added to the hydrolysis mixture. They can be added concomitantly, sequentially, continuously, semi-continuously, or batch wise. The amount of water added to the reaction mixture is sufficient to effectively dissolve the acid salts of long chain amino acid and alkylamine. The extractant solvent is selected on the same principle as described in the previous section for the extractant solvent for the hydrolysis solution with alkali hydroxide.

After dissolution and extraction according to the process of present invention, the aqueous phase containing the acid salts of long chain amino acid and alkylamine and the extractant solvent phase rich in long chain dibasic acid and short chain alkanoic acid are separated. An added advantage of the present invention is that all colored materials are transferred into organic extractant phase and the extracted aqueous solution of long chain amino acid and alkylamine is nearly colorless.

After phase separation, the organic extractant phase is treated in the same way as for the extractant phase obtained from acidification and extraction of the hydrolysis solution using alkali hydroxide.

The strongly acidic aqueous phase is neutralized with a basic agent to a neutral pH in the range of 5 to 9, more preferably 5 to 8, most preferably 6 to 7, to precipitate long chain amino acid. After cooling, the precipitated solid is isolated by means of solid-liquid separation, i.e., filtration or centrifuge.

The basic agent is selected from ammonia, alkali and ammonium salts of hydroxide, bicarbonate, carbonate, bisulfite, sulfite, and carboxylate. Preferably, the basic agent is alkali hydroxide or ammonium hydroxide, and most preferably, sodium hydroxide.

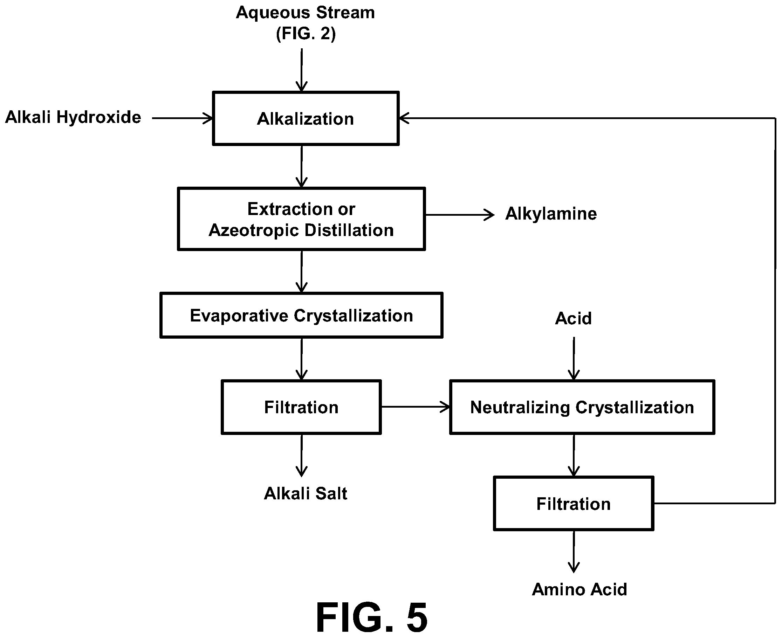

The mother liquor obtained after the isolation of long chain amino acid can be treated according to the scheme illustrated in FIG. 5 to separate alkylamine, inorganic salt, and to recover dissolved long chain amino acid.

When sodium hydroxide is used as the basic agent to neutralize sulfuric acid, more sodium hydroxide is added to the mother liquor to a basic pH, alkylamine can be extracted with an extractant solvent, or preferably by azeotropic distillation. After complete removal of alkylamine, the solution is further evaporated to separate inorganic salt, preferably, sodium sulfate. Long chain amino acids can be recovered by adding an acid to adjust the pH to neutral.

If ammonia or ammonium hydroxide is used as the basic agent to neutralize sulfuric acid, and after more basic agent is added to the mother liquor to a basic pH, alkylamine can only be recovered by extraction. Distillation of the basic solution will remove ammonia instead of alkylamine.

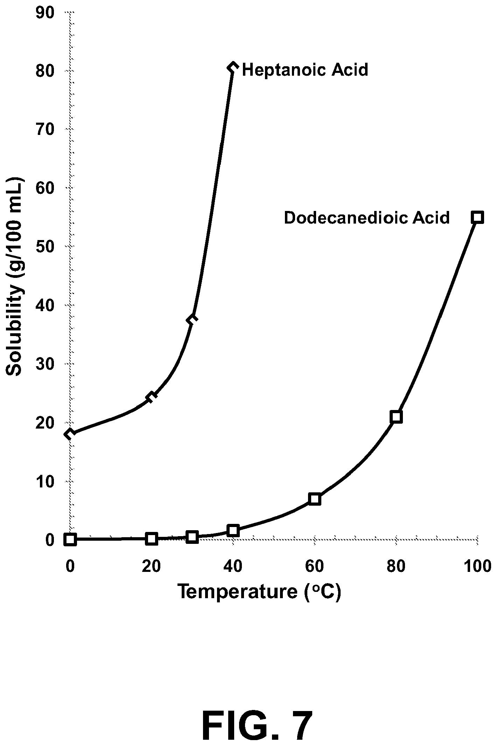

In an alkali hydroxide hydrolysis of the mixed amide derivatives, two mole equivalents of alkali hydroxide are required, but more than two equivalents, usually between 2.5 to 3 equivalents of alkali hydroxide are used to achieve a complete hydrolysis. The excess basic agent consumes additional acid and generate an extra amount of inorganic salt. The present inventor found that alkali salts of long chain amino acid and dibasic acid have little solubility at room temperature in water or in a solution of alkali hydroxide, as illustrated in FIG. 6 and FIG. 7, and can be crystallized from the hydrolysis solution. Upon cooling, a mixture of alkali salts of long chain dibasic acid and amino acid is precipitated. After separation of these insoluble salts, the excess alkali hydroxide can be recycled to the hydrolysis stage to reduce the use of alkali hydroxide. The recycling can be performed until the concentration of alkali salt of alkanoic acid becomes saturated, at which point, the mother liquor is purged from the hydrolysis stage and acidified with an acid to form alkanoic acid.

Precipitation of the alkali salts of long chain amino acid and dibasic acid can be carried out at temperature from 0 °C to 40 °C, preferably from 10 °C to 30 °C, most preferably from 15 °C to 25 °C. At this preferable temperature, alkali salts are precipitated in high yield.

Alternatively, the hydrolysis solution is cooled to precipitate alkali salts of long chain dibasic acid and amino acid, which are separated by means of a solid-liquid separation to provide a mother liquor. Alkylamine is then recovered from the mother liquor by distillation or extraction with an extractant solvent.

Precipitation of the alkali salts of long chain amino acid and dibasic acid is performed preferably after removal of alkylamine, but the precipitation can also be carried out before removing alkylamine, in fact, little difference is observed if alkylamine is not removed first.

Preferably, alkylamine is removed from the solution before precipitation, because the strongly alkaline hydrolysis solution makes alkylamine removal from the solution convenient to perform by distillation or by extraction with an extractant solvent.

Acidification of the mother liquor with an acid results in the formation of alkanoic acid. The alkanoic acid can be separated by phase separation, or it can be extracted with an extractant solvent. Alkanoic acid of required purity can be obtained by distillation.

The mixed alkali salts of long chain amino acid and dibasic acid can be used directly for the separation or they can first be converted to a mixture of long chain amino acid and dibasic acid before the separation. To prepare a mixture of long chain dibasic acid and amino acid, their alkali salts are dissolved or suspended in water and neutralized with an acid to a pH in the range from 4 to 5. The alkali salts are converted to a mixture of their respective long chain dibasic acid and amino acid.

A mixture of long chain amino acid and dibasic acid can be separated by selectively dissolving long chain dibasic acid in an aqueous solution of ammonia, ammonium hydroxide, ammonium bicarbonate, ammonium carbonate, alkylamines, or a mixture of two or more thereof. Upon removal of excess ammonia or alkylamine by heating, long chain dibasic acid is converted to the ammonium salt, which is soluble in water, while the amino acid is nearly insoluble. The long chain amino acid is recovered by means of solid-liquid filtration, while the long chain dibasic acid, dissolved in the mother liquor as the ammonium salt, is recovered by adding an acid to convert the ammonium salt to long chain dibasic acid.

The mixture of long chain amino acid and dibasic acid or their alkali salts can be separated into long chain amino acid and dibasic acid by first dissolving or suspending in water, adding an acid in the presence of an extractant solvent to form an aqueous solution of an acid salt of long chain amino acid and an extractant solvent phase containing long chain dibasic acid according to process disclosed in the present invention.

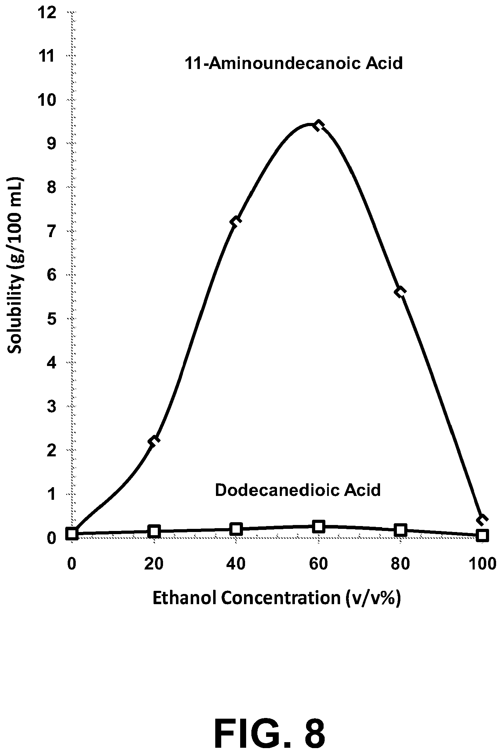

Although the alkali salts are nearly insoluble in water and organic solvents, the present inventor found that the solubility of the alkali salts of long chain amino acid and dibasic acid can be drastically altered by using an aqueous solvent. For example, FIG. 8 illustrates the solubility change of sodium salts of 11-aminoundecanoic acid and dodecanedioic acid in aqueous ethanol. Sodium salts of 11-aminoundecanoic acid and dodecanedioic acid are nearly insoluble in both water and ethanol, but aqueous ethanol drastically increases the solubility of sodium of 11-aminoundecanoic acid, while the sodium salt of dodecanedioic acid remains unchanged. This surprising finding renders the separation of these two salts possible.

Suitable solvents include methanol, ethanol, propanol, isopropanol, tert-butanol, n-butanol, isobutanol, sec-butanol, ethylene glycol, propylene glycol, diethylene glycol, glycerol, tetrahydrofuran, dioxane, morphine, N-methyl morphine, dimethyl formamide, dimethyl acetamide, N-methylpyrrolidone, tetramethylurea, and a mixture of two or more thereof. Preferable solvent is selected from one of the lower alcohols.

The most preferable solvent is ethanol.

The concentration of aqueous ethanol is from 20% to 90%, preferably from 40% to 80%, most preferably from 50% to 70%.

In order to separate the alkali salts of long chain amino acid and dibasic acid, the mixed salts can be stirred in an aqueous solution of selected solvent at a temperature from 0 °C to 40 °C, preferably from 15 °C to 25 °C from 30 minutes to 2 hours. Alkali salt of long chain amino acid dissolves while alkali salt of long chain dibasic acid remains as solid. More preferably, the mixed salts are heated in an aqueous solvent to dissolve, then cooled to crystallize the alkali salt of long chain dibasic acid.

The amount of an aqueous solvent is greater than the effective amount to dissolve alkali salt of long chain amino acid. Too large an amount is to be avoided as a small amount of alkali salt of long chain dibasic acid will dissolve and complicate the separation.

The insoluble alkali salt of long chain dibasic acid in an aqueous solvent is separated by means of solid-liquid separation from soluble alkali salt of long chain amino acid. Long chain dibasic acid is recovered by adding an acid to an aqueous suspension or solution of the alkali salt, then by means of solid-liquid separation.

The mother liquor after the separation of alkali salt of long chain dibasic acid is distilled to recover solvent and the aqueous solution of alkali salt of long chain amino acid is neutralized with an acid to a pH in the range from 5 to 9 to recover the long chain amino acid.

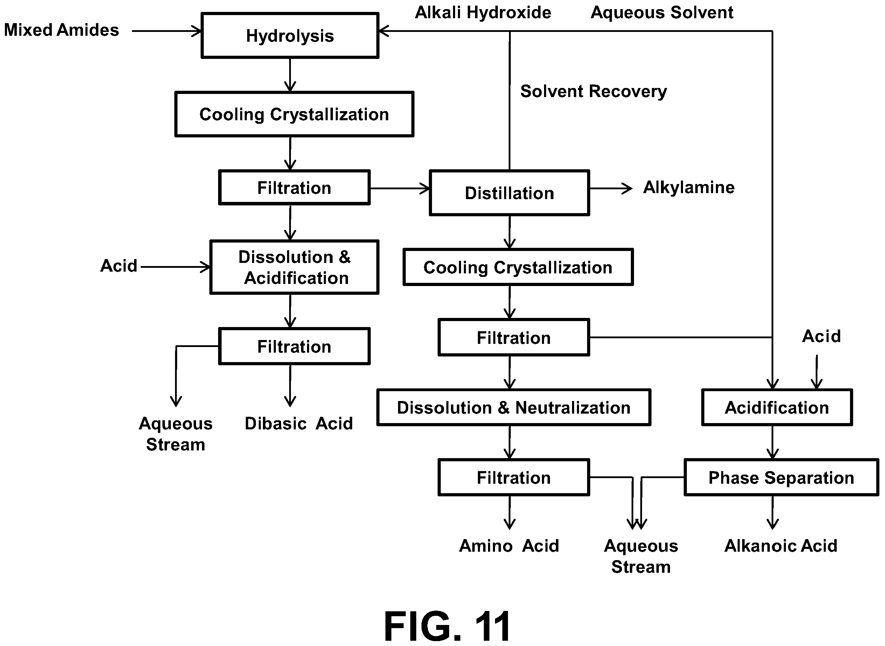

FIG. 11 demonstrates an integrated process for an alkali hydroxide hydrolysis of the mixed amide derivatives in an aqueous solvent, most preferably, ethanol, for a direct isolation of the alkali salt of long chain dibasic acid and the alkali salt of long chain amino acid without isolating their mixture.

After the hydrolysis is completed, the solution is cooled to crystallize the alkali salt of long chain dibasic acid. The crystallization takes place at a temperature in the range from 0 °C to 40 °C, more preferably from 15 °C to 25 °C. The crystallized salt of long chain dibasic acid is separated by means of solid-liquid separation, i.e., filtration or centrifuge, to provide a mother liquor.

The mother liquor is distilled first to recover solvent and then to remove alkylamine. The residual solution of distillation is then cooled to crystallize the alkali salt of long chain amino acid, which is separated by means of solid-liquid separation, i.e., filtration or centrifuge, to provide a mother liquor containing mainly alkali salt of alkanoic acid. The crystallization of the alkali salt of long chain amino acid takes place at a temperature in the range from 0 °C to 40 °C, more preferably from 15 °C to 25 °C.

The alkali salt of long chain dibasic acid and amino acid can be further purified by recrystallization in water or an aqueous solvent.

Long chain dibasic acid is obtained by dissolving or suspending the alkali salt in water, acidified to a pH in the range from 1 to 5, more preferably from 3-4, with an acid. After completion of crystallization, the crystalline solid is separated by means of solid-liquid separation.

Long chain amino acid is obtained by dissolving or suspending the alkali salt in water, which is then neutralized with an acid to a pH in the range from 5 to 9, more preferably in the range from 6 to 8.

The mother liquor containing the alkali salt of alkanoic acid also contains excess alkali hydroxide. This solution can be recycled to the hydrolysis stage to make use of the excess alkali hydroxide until the concentration of alkali salt of short chain alkanoic acid becomes saturated. The solution is then purged from the process and acidified with an acid to yield alkanoic acid, which can be isolated by a phase separation or by extraction with an extractant solvent.

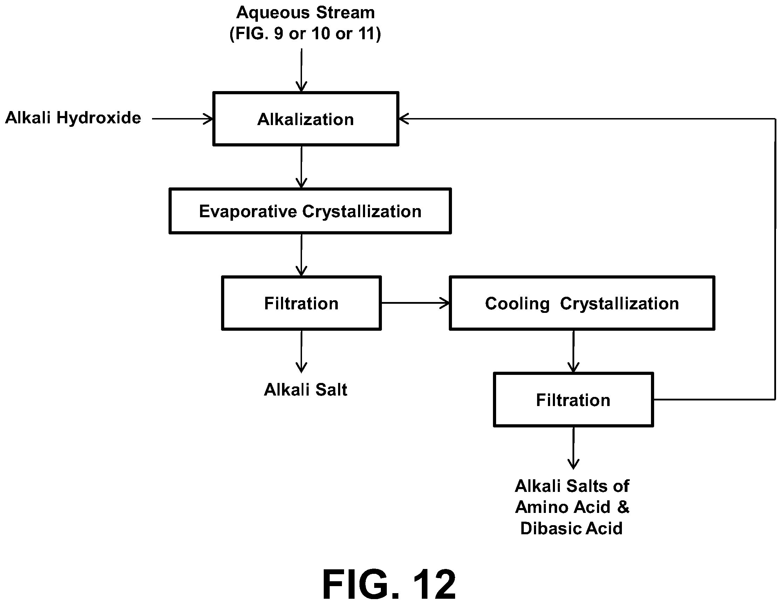

The aqueous stream, generated as waste water after the separation of long chain amino acid, dibasic acid, or alkanoic acid, contains inorganic salt, most preferably sodium sulfate, if sulfuric acid and sodium hydroxide are selected as the most preferable acid and basic agent respectively, is treated according a scheme illustrated in FIG. 12 to isolate alkali salt and to recover alkali salts of long chain amino acid and dibasic acid. An alkali hydroxide is introduced to an aqueous stream to increase the solubility of alkali salts of long chain amino acid and dibasic acid at higher temperature so as to facilitate the isolation of alkali salt by evaporative crystallization. The mother liquor is then cooled to precipitate a mixture of alkali salts of long chain dibasic acid and amino acid, which is separated by means of solid-liquid separation. The mother liquor is returned to the beginning step to complete a cycle. This cyclic process ensures that no waste, other than inorganic salt, will be discharged from the process.

The process according to the present invention achieves a complete separation of each component in the production of long chain amino acids and dibasic acids without discharging any waste aqueous stream from the process.

### EXAMPLES

The following examples illustrate the practice of this invention but are not intended to limit its scope.

### REFERENCE EXAMPLE 1

This example relates to the separation of 11-aminoundecanoic acid, dodecanedioic acid, hexylamine, heptanoic acid, and stearic acid from their mixture obtained from sodium hydroxide hydrolysis of the mixed amide derivatives.

A mixture of the starting solution was obtained by hydrolyzing 150 g of the mixed amide derivatives prepared from methyl 12-ketostearate according to WO2017/088218 with 60 g of sodium hydroxide in 800 mL of water.

The solution was azeotropically distilled with a 2.5x30 cm vacuum jacketed column packed with porcelain berl saddles, first to obtain methanol, then an azeotrope of hexylamine-water until the pH of the overhead became neutral at a pH of 7-8. The distillate was separated into two phases and the lower aqueous phase was continuously returned to the distillation flask. The crude hexylamine was dehydrated by azeotropic distillation to yield 20.5 g of hexylamine.

To the residual solution were added 800 mL of toluene, followed by 100 g of sulfuric acid. The mixture was vigorously stirred for 60 minutes at 85 °C and transferred to a separatory funnel to separate the aqueous phase. The dark-colored upper toluene phase was washed with hot deionized water and the washing was combined with the aqueous phase.

To the colorless aqueous phase was added 1.0 g of activated carbon and stirred at 80 °C for 45 minutes and the solution was filtrated to obtain a clear, colorless solution. The solution was neutralized with a solution of sodium hydroxide to a pH of 7.5 at about 70 °C to yield a crystalline suspension. After cooling to 35 °C, the suspension was filtrated and the solid material washed three times with deionized water. After drying, 42.5 g of white 11-aminoundecanoic acid was obtained.

To the mother liquor of about 1200 mL was added 1.0 g of sodium hydroxide. The solution was concentrated and sodium sulfate removed by filtration three times so that 300 mL of solution remained. This basic solution was adjusted with dilute sulfuric acid to a pH of 7.5 to recover another 0.8 g of 11-aminoundecanoic acid.

The toluene solution was washed with hot deionized water once and cooled on ice to 5 °C to obtain a crystalline suspension. After filtration, washing with cold toluene, and drying, 45.2 g of dodecanedioic acid was obtained. The product was off-white.

The toluene filtrate was combined with toluene washing and distilled to recover toluene. The residual was then vacuum distilled with a short path column to yield 24.5 g of heptanoic acid.

The residual after distillation was black and weighted 26.6 g. The residual was mixed with 200 mL of methanol and added 1.0 g of sulfuric acid. After the mixture was refluxed for 2 hours and sulfuric acid was neutralized with sodium methoxide. Methanol was removed by distillation and the residual methyl esters were distilled to obtain a mixture of colorless methyl esters, of which 80% was methyl stearate, 5% was methyl heptanoate, 15% was dimethyl dodecanedioate. About 1.5 g of black residual remained in the distillation flask.

### REFERENCE EXAMPLE 2

This example relates to the separation of 11-aminoundecanoic acid, dodecanedioic Acid, hexylamine, heptanoic acid, and stearic acid from their mixture of sulfuric acid hydrolysis of the mixed amide derivatives .

A mixture of the starting suspension was obtained by hydrolyzing 150 g of the mixed amide derivatives prepared from methyl 12-ketostearate according to WO2017/088218 with a mixture of 150 g of sulfuric acid and 30 g of water. During the hydrolysis, low-boiling methanol was continuously removed.

To the reaction suspension were added 800 g of water and 800 mL of toluene. The mixture was vigorously stirred for 60 minutes at 85 °C and transferred to a separatory funnel to separate the two phases.

The toluene phase was treated the same way as in Example 1 and similar results were obtained for each component.

The aqueous phase was neutralized with aqueous solution of sodium hydroxide to neutral pH at 7.5 for a total of 115 g of sodium hydroxide. After cooling to 35 °C, the crystalline solid was filtered off, washed three times with deionized water, dried to yield 41.6 g of 11-aminoundecanoic acid.

To the mother liquor was added an additional solution of sodium hydroxide containing 20 g of sodium hydroxide. The solution was azeotropically distilled with a 2.5x30 cm vacuum jacketed column filled with porcelain berl saddles until the pH of the overhead became a pH of 7-8. The distillate is separated into two phases and the lower aqueous phase was continuously returned to the distillation flask. The crude hexylamine was dehydrated by azeotropic distillation to yield 21.5 g of hexylamine.

After hexylamine was completely removed, the solution containing sodium sulfate was treated the same way as in Example 1. An additional 1.2 g of 11-aminoundecanoic acid was recovered from the mother liquor.

### REFERENCE EXAMPLE 3

This example relates to the separation of 9-aminononanoic acid, sebacic acid, octylamine, and pelargonic acid.

A mixture of the starting solution was obtained by hydrolyzing 150 g of the mixed amide derivatives prepared from methyl 10-ketostearate according to WO2017/088218 with 60 g of sodium hydroxide in 800 mL of water.

To the turbid solution was added 200 mL of toluene and the mixture was vigorously stirred at a temperature of 80 °C for 45 minutes. Afterwards, the toluene phase was separated and removed to yield a residual, which was distilled to obtain 29.5 g of n-octylamine.

The aqueous phase was treated the same way as in Example 1 to obtain 36.9 g of pelargonic acid, 39.6 g of 9-aminononanoic acid, and 45.5 g of sebacic acid.

### REFERENCE EXAMPLE 4

This example relates to the separation of 13-aminotridecanoic acid, brassylic acid, hexylamine, and heptanoic acid.

A mixture of the starting solution was obtained by hydrolyzing 180 g of the mixed amide derivatives prepared from methyl ester of 14-ketoarachidic acid according to WO2017/088218 with 60 g of sodium hydroxide in 800 mL of water.

The reaction solution was treated the same way as in Example 1 to yield 23.5 g of hexylamine, 29.4 g of heptanoic acid, 53.1 g of 13-aminotridecanoic acid, and 62.9 g of brassylic acid.

### EXAMPLE 5

This example relates to the separation of 11-aminoundecanoic acid, dodecanedioic acid, hexylamine, heptanoic acid, and stearic acid by the method of co-precipitation of sodium salts of 11-aminoundecanoic acid and dodecanedioic acid.

A mixture of the starting solution was obtained by hydrolyzing 150 g of the mixed amide derivatives prepared from methyl 12-ketostearate according to WO2017/088218 with 60 g of sodium hydroxide in 800 mL of water.

The solution was azeotropically distilled with a 2.5x30 cm vacuum jacketed column packed with porcelain berl saddles, first to obtain methanol, then an azeotrope of hexylamine-water until the pH of the overhead became neutral at a pH of 7-8. The distillate was separated into two phases and the lower aqueous phase was continuously returned to the distillation flask. The crude hexylamine was dehydrated by azeotropic distillation to yield 20.5 g of hexylamine.

To the residual solution of distillation was added 500 mL of water. The solution was slowly stirred while cooling to room temperature. The precipitate was then filtered and washed with cold water to give a mixture of the alkali salts of 11-aminoundecanoic acid and dodecanedioic acid. Half of the mother liquor is recycled to the hydrolysis stage. The other half is concentrated to 300 mL and acidified with sulfuric acid to yield an oily layer of heptanoic acid.

The solid material was dissolved in 500 mL of 65% aqueous ethanol by heating and then slowed cooled to room temperature to crystallize sodium salt of dodecanedioic acid. After filtration and washing with water, the mother liquor is distilled to recover ethanol and the aqueous solution was neutralized with sulfuric acid to a pH of 6-7 to yield 41.4 g of 11-aminoundecanoic acid.

The sodium salt of dodecanedioic acid was suspended in 400 mL of water and acidified with sulfuric acid to yield 46.3 g of dodecanedioic acid.

### REFERENCE EXAMPLE 6

This example relates to the sodium hydroxide hydrolysis of the mixed amide derivatives in aqueous ethanol and the separation of 11-aminoundecanoic acid, dodecanedioic acid, hexylamine, and heptanoic acid.

A mixture of the starting solution was obtained by hydrolyzing 160 g of the mixed amide ethyl ester prepared from ethyl 10-ketostearate according to WO2017/088218 with 60 g of sodium hydroxide in 800 mL of of 65% aqueous ethanol.

The solution after hydrolysis was cooled slowly to room temperature and the crystallized sodium salt of dodecanedioic acid was filtered and washed with dilute aqueous ethanol. The solid material was suspended in 300 mL of water, to which was added sufficient sulfuric acid to a pH of 2. The solid material was filtered and washed three times with deionized water to give 46.8 g of dodecanedioic acid.

The alcoholic mother liquor was distilled with a 2.5x30 cm vacuum jacketed column packed with porcelain berl saddles, first to obtain ethanol, then an azeotrope of hexylamine-water until the pH of the overhead became neutral at a pH of 7-8. The distillate was separated into two phases and the lower aqueous phase was continuously returned to the distillation flask. The crude hexylamine was dehydrated by azeotropic distillation to yield 21.4 g of hexylamine.

To the residual solution of ethanol distillation was added 400 mL of water. The solution was slowly stirred while being cooled to room temperature to crystallize sodium salt of 11-aminoundecanoic acid. After filtration and washing with water, the solid was suspended in 200 mL of water and neutralized with sulfuric acid to a pH of 7.5. Filtration, washing with deionized water, and drying, yield 40.9 g of 11-aminoundecanoic acid.

The mother liquor after separating the sodium salt of 11-aminoundecanoic acid was concentrated to a volume of 300 mL, and acidified with sulfuric acid to give an oily upper layer of heptanoic acid, which was separated by phase separation.

The aqueous streams were combined and added 2 g of sodium hydroxide. The solution were boiling to crystallize sodium sulfate, which was filtered at 80 °C and washed with hot saturated solution of sodium sulfate. The mother is then cooled slowly to 35 °C to crystallize sodium salt of 11-aminoundecanoic acid and dodecanedioic acid, which was recovered by filtration.

## Claims

1. A process for the separation of long chain amino acid, long chain dibasic acid, alkylamine, and alkanoic acid of the following structures: 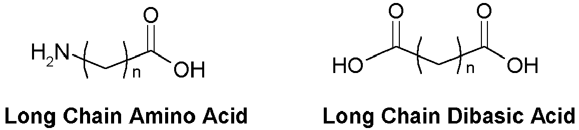 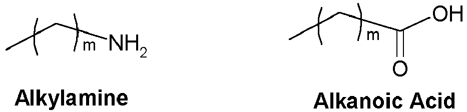 from at least two of them in an alkali hydroxide hydrolysis mixture of mixed amide derivatives of the following structures: 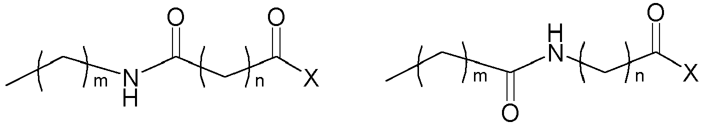 wherein m is an integer from 0 to 10;
n is an integer from 6 to 20;
X is OR or NR₁R₂, wherein OR is OH, Ci-Cs monohydric alcohol, or Ci-Cs polyhydric alcohol, and R₁ and R₂ are each independently hydrogen or Ci-Cs alkyl group; the process comprising:
(1) recovering the alkylamine from an aqueous solution of the alkali hydroxide hydrolysis mixture of the mixed amide derivatives by distilling or by extracting with an extractant solvent;
(2) cooling the aqueous solution of step (1) to precipitate a mixture of alkali salts of the long chain amino acid and long chain dibasic acid;
(3) separating the mixture of the alkali salts of long chain amino acid and long chain dibasic acid by means of solid-liquid separation to provide a mother liquor;
(4) separating each of the long chain amino acid and long chain dibasic acid from the mixture of the alkali salts of the long chain amino acid and long chain dibasic acid, by acidification-extraction of the long chain dibasic acid with an extractant solvent, or by selective dissolution of the alkali salt of the long chain amino acid in an aqueous solvent, or by selective dissolution of the long chain dibasic acid in an aqueous solution of ammonium hydroxide, ammonium bicarbonate, or ammonium carbonate or their mixture; and
(5) adding an acid to the mother liquor of step (3) to obtain the alkanoic acid.

2. The process according to claim 1, wherein the step (4) of separating each of the long chain amino acid and the long chain dibasic acid comprises:
(a) adding an acid to an aqueous solution or suspension of the mixture of the alkali salts of the long chain amino acid and long chain dibasic acid to a pH in the range from 4 to 5 to obtain a mixture of the long chain amino acid and long chain dibasic acid;
(b) separating the mixture of the long chain amino acid and long chain dibasic acid by means of solid-liquid separation;
(c) suspending the mixture of the long chain amino and long chain dibasic acid in an aqueous solution of ammonia, ammonium hydroxide, ammonium bicarbonate, ammonium carbonate, or their mixture to convert the long chain dibasic acid to its ammonium salt;
(d) separating the long chain amino acid by means of solid-liquid separation to provide a mother liquor; and
(e) adding an acid to the mother liquor of step (d) to precipitate the long chain dibasic acid.

3. The process according to claim 1, wherein the step (4) of separating each of the long chain amino acid and long chain dibasic acid comprises:
(a) adding an acid to an aqueous suspension or solution of the mixture of the alkali salts of the long chain amino acid and long chain dibasic acid in the presence of an extractant solvent;
(b) separating the mixture of step (a) into an aqueous phase containing the acid salt of the long chain amino acid and an extractant phase containing the long chain dibasic acid;
(c) neutralizing the aqueous phase of step (b) with a basic agent to yield the long chain amino acid;
(d) cooling the extractant phase to crystallize the long chain dibasic acid; and
(e) separate the long chain dibasic acid by means of solid-liquid separation.

4. The process according to claim 1, wherein the step (4) of separating each of the long chain amino acid and long chain dibasic acid comprises:
(a) suspending or dissolving the mixture of the alkali salts of the long chain amino acid and long chain dibasic acid in an aqueous solvent;
(b) cooling to crystallize the alkali salt of the long chain dibasic acid;
(c) separating the precipitate of the alkali salt of the long chain dibasic acid by means of solid-liquid separation to provide a mother liquor;
(d) adding an acid to an aqueous suspension or solution of the precipitate of step (c) to acidic pH to yield the long chain dibasic acid; and
(e) distilling the mother liquor of step (c) to recover the solvent and adding an acid to neutralize the solution to yield the long chain amino acid.

5. The process as claimed in claim 4, wherein the aqueous solvent in step (a) is selected from the group consisting of aqueous methanol, ethanol, propanol, isopropanol, tert-butanol, n-butanol, isobutanol, sec-butanol, ethylene glycol, propylene glycol, diethylene glycol, glycerol, tetrahydrofuran, dioxane, morphine, N-methylmorphine, dimethylformamide, dimethyl acetamide, N-methylpyrrolidone, tetramethylurea, and a mixture of two or more thereof.

6. The process as claimed in claim 4, wherein the step (a) is performed in an aqueous ethanol containing from 20% to 90% of ethanol.

7. The process according to claim 1, wherein the acid used in the step (5) is selected from the group consisting of hydrochloric acid, hydrobromic acid, hydroiodic acid, sulfuric acid, nitric acid, phosphoric acid, methanesulfonic acid, ethanesulfonic acid, propanesulfonic acid, isethionic acid, benzenesulfonic acid, toluenesulfonic acid, xylenesulfonic acid, sulfamic acid, malic acid, maleic acid, tartaric acid, glycolic acid, lactic acid, citric acid, oxalic acid, formic acid, acetic acid, propionic acid, and a mixture of two or more thereof.

8. The process according to claim 1, wherein the extractant solvent used in the step (1) is selected from the group consisting of butyl formate, isobutyl formate, butyl acetate, isobutyl acetate, propyl acetate, isopropyl acetate, ethyl acetate, ethyl propionate, octyl acetate, benzene, toluene, xylene, cumene, anisole, diethyl ether, diisopropyl ether, dibutyl ether, methyl tert-butyl ether, ethyl tert-butyl ether, methyl tetrahydrofuran, petroleum ether, cyclohexane, dichloroethane, methylene chloride, chloroform, carbon tetrachloride, trifluoromethylbenzene, n-butanol, isobutanol, amyl alcohol, isoamyl alcohol, hexanol, cyclohexanol, 2-ethylhexanol, isooctanol, sec-octanol, butanone, pentanone, hexanone, cyclohexanone, methyl isobutyl ketone, and a mixture of two or more thereof.

9. The process according to claim 1, wherein the long chain amino acid is 9- aminononanoic acid, 11-aminoundecanoic acid, or 13- aminotridecanoic acid.

10. The process according to claim 1, wherein the long chain amino acid is 11- aminoundecanoic acid.

11. The process according to claim 1, wherein the long chain dibasic acid is sebacic acid, dodecanedioic acid, or brassylic acid.

12. The process according to claim 1, wherein the long chain dibasic acid is dodecanedioic acid.

13. The process according to claim 1, wherein the short chain alkanoic acid is heptanoic acid.

14. The process according to claim 1, wherein the alkylamine is n-hexylamine.

## Patentansprüche

1. Prozess zum Trennen einer langkettigen Aminosäure, einer langkettigen zweibasigen Säure, Alkylamin und Alkansäure der folgenden Strukturen: 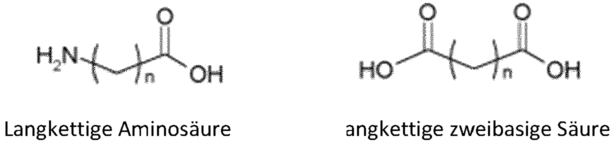 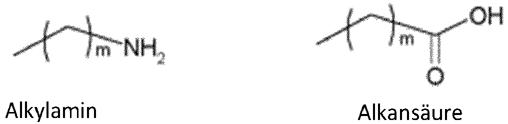 aus mindestens zwei davon in einem Alkalihydroxid-Hydrolysegemisch aus gemischten Amidderivaten der folgenden Strukturen: 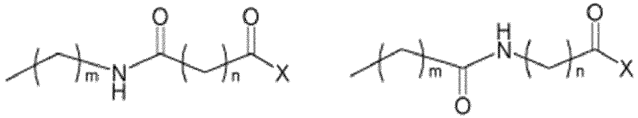 wobei m eine ganze Zahl von 0 bis 10 ist;
n eine ganze Zahl von 6 bis 20 ist;
X OR oder NR₁R₂ ist, wobei OR OH, einwertiger C₁-C₈-Alkohol oder mehrwertiger C₁-C₈-Alkohol ist und R₁ und R₂ jeweils unabhängig voneinander Wasserstoff oder eine C₁-C₈-Alkylgruppe sind; wobei der Prozess umfasst:
(1) Gewinnen des Alkylamins aus einer wässrigen Lösung des Alkalihydroxid-Hydrolysegemisches aus gemischten Amidderivaten durch Destillation oder durch Extrahieren mit einem Extraktionslösungsmittel;
(2) Abkühlen der wässrigen Lösung aus Schritt (1), um ein Gemisch aus Alkalisalzen der langkettigen Aminosäure und der langkettigen zweibasigen Säure auszufällen;
(3) Abscheiden des Gemisches aus den Alkalisalzen einer langkettigen Aminosäure und langkettigen zweibasigen Säure mittels Fest-Flüssig-Abscheidung, um eine Mutterlauge bereitzustellen;
(4) Abscheiden der langkettigen Aminosäure und einer langkettigen zweibasigen Säure von dem Gemisch der Alkalisalze der langkettigen Aminosäure und der langkettigen zweibasigen Säure durch Ansäuern-Extrahieren der langkettigen zweibasigen Säure mit einem Extraktionslösungsmittel oder durch selektives Auflösen des Alkalisalzes der langkettigen Aminosäure in einem wässrigen Lösungsmittel oder durch selektives Auflösen der langkettigen zweibasigen Säure in einer wässrigen Lösung aus Ammoniumhydroxid, Ammoniumbicarbonat oder Ammoniumcarbonat oder deren Gemisch; und
(5) Zugeben einer Säure zur Mutterlauge aus Schritt (3), um die Alkansäure zu erhalten.

2. Prozess nach Anspruch 1, wobei der Schritt (4) des Abscheidens von jeder der langkettigen Aminosäure und der langkettigen zweibasigen Säure umfasst:
(a) Zugeben einer Säure zu einer wässrigen Lösung oder Suspendieren des Gemisches aus den Alkalisalzen der langkettigen Aminosäure und der langkettigen zweibasigen Säure bis zu einem pH-Wert im Bereich von 4 bis 5, um ein Gemisch aus der langkettigen Aminosäure und der langkettigen zweibasigen Säure zu erhalten;
(b) Abscheiden des Gemisches aus der langkettigen Aminosäure und der langkettigen zweibasigen Säure mittels Fest-Flüssig-Abscheidung;
(c) Suspendieren des Gemisches aus der langkettigen Aminosäure und der langkettigen zweibasigen Säure in einer wässrigen Lösung aus Ammoniak, Ammoniumhydroxid, Ammoniumbicarbonat, Ammoniumcarbonat oder deren Gemisch, um die langkettige zweibasige Säure in ihr Ammoniumsalz umzuwandeln;
(d) Abscheiden der langkettigen Aminosäure mittels Fest-Flüssig-Abscheidung, um eine Mutterlauge bereitzustellen; und
(e) Zugeben einer Säure zu der Mutterlauge aus Schritt (d), um die langkettige zweibasige Säure auszufällen.

3. Prozess nach Anspruch 1, wobei der Schritt (4) des Abscheidens von jeder der langkettigen Aminosäure und der langkettigen zweibasigen Säure umfasst:
(a) Zugeben einer Säure zu einer wässrigen Suspension oder Lösung des Gemisches aus den Alkalisalzen der langkettigen Aminosäure und der langkettigen zweibasigen Säure in der Gegenwart eines Extraktionslösungsmittels;
(b) Abscheiden des Gemisches aus Schritt (a) in eine wässrige Phase, die das Säuresalz der langkettigen Aminosäure enthält, und eine Extraktionsphase, welche die langkettige zweibasige Säure enthält;
(c) Neutralisieren der wässrigen Phase aus Schritt (b) mit einem basischen Mittel, um die langkettige Aminosäure zu ergeben;
(d) Abkühlen der Extraktionsphase, um die langkettige zweibasige Säure zu kristallisieren; und
(e) Abscheiden der langkettigen zweibasigen Säure mittels Fest-Flüssig-Abscheidung.

4. Prozess nach Anspruch 1, wobei der Schritt (4) des Abscheidens von jeder der langkettigen Aminosäure und der langkettigen zweibasigen Säure umfasst:
(a) Suspendieren oder Auflösen des Gemisches aus den Alkalisalzen der langkettigen Aminosäure und der langkettigen zweibasigen Säure in einem wässrigen Lösungsmittel;
(b) Abkühlen, um das Alkalisalz der langkettigen zweibasigen Säure zu kristallisieren;
(c) Abscheiden der Ausfällung des Alkalisalzes der langkettigen zweibasigen Säure mittels Fest-Flüssig-Abscheidung, um eine Mutterlauge bereitzustellen;
(d) Zugeben einer Säure zu einer wässrigen Suspension oder Lösung der Ausfällung aus Schritt (c) bis zu einem sauren pH-Wert, um die langkettige zweibasige Säure zu ergeben; und
(e) Destillieren der Mutterlauge aus Schritt (c), um das Lösungsmittel zurückzugewinnen, und Zugeben einer Säure, um die Lösung zu neutralisieren, um die langkettige Aminosäure zu ergeben.

5. Prozess nach Anspruch 4, wobei das wässrige Lösungsmittel in Schritt (a) aus der Gruppe ausgewählt ist, bestehend aus wässrigem Methanol, Ethanol, Propanol, Isopropanol, tert-Butanol, n-Butanol, Isobutanol, sec-Butanol, Ethylenglykol, Propylenglykol, Diethylenglykol, Glycerin, Tetrahydrofuran, Dioxan, Morphin, N-Methylmorphin, Dimethylformamid, Dimethylacetamid, N-Methylpyrrolidon, Tetramethylharnstoff und einem Gemisch aus zwei oder mehreren davon.

6. Prozess nach Anspruch 4, wobei der Schritt (a) in einer wässrigen Ethanollösung durchgeführt wird, die 20 % bis 90 % Ethanol enthält.

7. Prozess nach Anspruch 1, wobei die Säure, die in Schritt (5) verwendet wird, aus der Gruppe ausgewählt ist, bestehend aus Salzsäure, Bromwasserstoffsäure, lodwasserstoffsäure, Schwefelsäure, Salpetersäure, Phosphorsäure, Methansulfonsäure, Ethansulfonsäure, Propansulfonsäure, Isethionsäure, Benzolsulfonsäure, Toluolsulfonsäure, Xylolsulfonsäure, Sulfaminsäure, Apfelsäure, Maleinsäure, Weinsäure, Glykolsäure, Milchsäure, Zitronensäure, Oxalsäure, Ameisensäure, Essigsäure, Propionsäure, oder einem Gemisch aus zwei oder mehreren davon.

8. Prozess nach Anspruch 1, wobei das Extraktionslösungsmittel, das in Schritt (1) verwendet wird, aus der Gruppe ausgewählt ist, bestehend aus Butylformiat, Isobutylformiat, Butylacetat, Isobutylacetat, Propylacetat, Isopropylacetat, Ethylacetat, Ethylpropionat, Octylacetat, Benzol, Toluol, Xylol, Cumol, Anisol, Diethylether, Diisopropylether, Dibutylether, Methyl-tert-butylether, Ethyl-tert-butylether, Methyltetrahydrofuran, Petrolether, Cyclohexan, Dichlorethan, Methylenchlorid, Chloroform, Tetrachlorkohlenstoff, Trifluormethylbenzol, n-Butanol, Isobutanol, Amylalkohol, Isoamylalkohol, Hexanol, Cyclohexanol, 2-Ethylhexanol, Isooctanol, sec-Octanol, Butanon, Pentanon, Hexanon, Cyclohexanon, Methylisobutylketon, oder einem Gemisch aus zwei oder mehreren davon.

9. Prozess nach Anspruch 1, wobei die langkettige Aminosäure 9-Aminononansäure, 11-Aminoundecansäure oder 13-Aminotridecansäure ist.

10. Prozess nach Anspruch 1, wobei die langkettige Aminosäure 11-Aminoundecansäure ist.

11. Prozess nach Anspruch 1, wobei die langkettige zweibasige Säure, Sebacinsäure, Dodecandisäure oder Brassylsäure ist.

12. Prozess nach Anspruch 1, wobei die langkettige zweibasige Säure Dodecandisäure ist.

13. Prozess nach Anspruch 1, wobei die kurzkettige Alkansäure Heptansäure ist.

14. Prozess nach Anspruch 1, wobei das Alkylamin n-Hexylamin ist.

## Revendications

1. Processus de séparation d'acide aminé à chaîne longue, d'acide dibasique à chaîne longue, d'alkylamine et d'acide alcanoïque des structures suivantes : 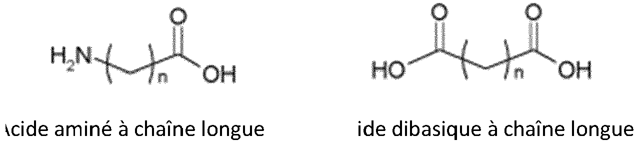 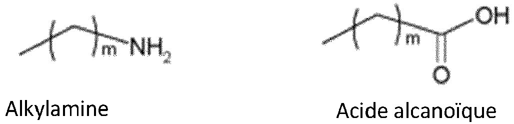 d'au moins deux d'entre eux dans un mélange d'hydrolyse d'hydroxyde alcalin de dérivés amides mixtes des structures suivantes : 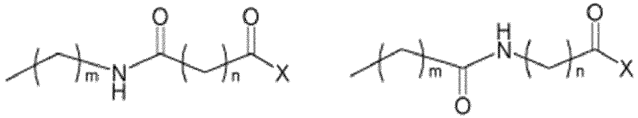 dans lequel m est un nombre entier compris entre 0 et 10 ;
n est un nombre entier compris entre 6 et 20 ;
X est OR ou NR₁R₂, dans lequel OR est OH, un alcool monohydrique en C₁-C₈ ou un alcool polyhydrique en C₁-C₈, et R₁ et R₂ sont chacun indépendamment un hydrogène ou un groupe alkyle en C₁-C₈; le processus comprenant:
(1) la récupération de l'alkylamine à partir d'une solution aqueuse du mélange d'hydrolyse d'hydroxyde alcalin des dérivés amides mixtes par distillation ou par extraction avec un solvant d'extraction ;
(2) le refroidissement de la solution aqueuse de l'étape (1) pour précipiter un mélange de sels alcalins de l'acide aminé à chaîne longue et de l'acide dibasique à chaîne longue ;
(3) la séparation du mélange des sels alcalins d'acide aminé à chaîne longue et d'acide dibasique à chaîne longue au moyen d'une séparation solide-liquide pour fournir une liqueur mère ;
(4) la séparation de chacun de l'acide aminé à chaîne longue et de l'acide dibasique à chaîne longue du mélange des sels alcalins de l'acide aminé à chaîne longue et de l'acide dibasique à chaîne longue, par acidification-extraction de l'acide dibasique à chaîne longue avec un solvant d'extraction, ou par dissolution sélective du sel alcalin de l'acide aminé à chaîne longue dans un solvant aqueux, ou par dissolution sélective de l'acide dibasique à chaîne longue dans une solution aqueuse d'hydroxyde d'ammonium, de bicarbonate d'ammonium ou de carbonate d'ammonium ou un mélange de ceux-ci ; et
(5) l'ajout d'un acide à la liqueur mère de l'étape (3) pour obtenir l'acide alcanoïque.

2. Processus selon la revendication 1, dans lequel l'étape (4) de séparation de chacun de l'acide aminé à chaîne longue et de l'acide dibasique à chaîne longue comprend :
(a) l'ajout d'un acide à une solution ou une suspension aqueuse du mélange des sels alcalins de l'acide aminé à chaîne longue et de l'acide dibasique à chaîne longue à un pH compris entre 4 et 5 pour obtenir un mélange de l'acide aminé à chaîne longue et de l'acide dibasique à chaîne longue ;
(b) la séparation du mélange de l'acide aminé à chaîne longue et de l'acide dibasique à chaîne longue au moyen d'une séparation solide-liquide ;
(c) la mise en suspension du mélange de l'acide aminé à chaîne longue et de l'acide dibasique à chaîne longue dans une solution aqueuse d'ammoniaque, d'hydroxyde d'ammonium, de bicarbonate d'ammonium, de carbonate d'ammonium, ou un mélange de ceux-ci pour convertir l'acide dibasique à chaîne longue en son sel d'ammonium ;
(d) la séparation de l'acide aminé à chaîne longue au moyen d'une séparation solide-liquide pour fournir une liqueur mère ; et
(e) l'ajout d'un acide à la liqueur mère de l'étape (d) pour précipiter l'acide dibasique à chaîne longue.

3. Processus selon la revendication 1, dans lequel l'étape (4) de séparation de chacun de l'acide aminé à chaîne longue et de l'acide dibasique à chaîne longue comprend :
(a) l'ajout d'un acide à une suspension ou une solution aqueuse du mélange des sels alcalins de l'acide aminé à chaîne longue et de l'acide dibasique à chaîne longue en présence d'un solvant d'extraction ;
(b) la séparation du mélange de l'étape (a) en une phase aqueuse contenant le sel d'acide de l'acide aminé à chaîne longue et une phase d'agent d'extraction contenant l'acide dibasique à chaîne longue ;
(c) la neutralisation de la phase aqueuse de l'étape (b) avec un agent basique pour obtenir l'acide aminé à chaîne longue ;
(d) le refroidissement de la phase d'agent d'extraction pour cristalliser l'acide dibasique à chaîne longue ; et
(e) la séparation de l'acide dibasique à chaîne longue au moyen d'une séparation solide-liquide.

4. Processus selon la revendication 1, dans lequel l'étape (4) de séparation de chacun de l'acide aminé à chaîne longue et de l'acide dibasique à chaîne longue comprend :
(a) la suspension ou la dissolution du mélange des sels alcalins de l'acide aminé à chaîne longue et de l'acide dibasique à chaîne longue dans un solvant aqueux ;
(b) le refroidissement pour cristalliser le sel alcalin de l'acide dibasique à chaîne longue ;
(c) la séparation du précipité du sel alcalin de l'acide dibasique à chaîne longue au moyen d'une séparation solide-liquide pour obtenir une liqueur mère ;
(d) l'ajout d'un acide à une suspension ou une solution aqueuse du précipité de l'étape (c) à un pH acide pour obtenir l'acide dibasique à chaîne longue ; et
(e) la distillation de la liqueur mère de l'étape (c) pour récupérer le solvant et ajouter un acide pour neutraliser la solution afin d'obtenir l'acide aminé à chaîne longue.

5. Processus selon la revendication 4, dans lequel le solvant aqueux dans l'étape (a) est sélectionné dans le groupe consistant en le méthanol aqueux, l'éthanol, le propanol, l'isopropanol, le tert-butanol, le n-butanol, l'isobutanol, le sec-butanol, l'éthylène glycol, le propylène glycol, le diéthylène glycol, le glycérol, le tétrahydrofurane, le dioxane, la morphine, la N-méthylmorphine, le diméthylformamide, le diméthylacétamide, la N-méthylpyrrolidone, la tétraméthylurée et un mélange de deux ou plus de ceux-ci.

6. Processus selon la revendication 4, dans lequel l'étape (a) est réalisée dans un éthanol aqueux contenant de 20 % à 90 % d'éthanol.

7. Processus selon la revendication 1, dans lequel l'acide utilisé à l'étape (5) est sélectionné dans le groupe consistant en l'acide chlorhydrique, l'acide bromhydrique, l'acide iodhydrique, l'acide sulfurique, l'acide nitrique, l'acide phosphorique, l'acide méthanesulfonique, l'acide éthanesulfonique, l'acide propanesulfonique, l'acide iséthionique, l'acide benzènesulfonique, l'acide toluènesulfonique, l'acide xylènesulfonique, l'acide sulfamique, l'acide malique, l'acide maléique, l'acide tartrique, l'acide glycolique, l'acide lactique, l'acide citrique, l'acide oxalique, l'acide formique, l'acide acétique, l'acide propionique ou un mélange de deux ou plus de ceux-ci.

8. Processus selon la revendication 1, dans lequel le solvant d'extraction utilisé à l'étape (1) est sélectionné dans le groupe consistant en le formiate de butyle, le formiate d'isobutyle, l'acétate de butyle, l'acétate d'isobutyle, l'acétate de propyle, l'acétate d'isopropyle, l'acétate d'éthyle, le propionate d'éthyle, l'acétate d'octyle, le benzène, le toluène, le xylène, le cumène, l'anisole, l'éther diéthylique, l'éther diisopropylique, l'éther dibutylique, l'éther tert-butylique méthylique, l'éther tert-butylique éthylique, le tétrahydrofurane méthylique, l'éther de pétrole, le cyclohexane, le dichloroéthane, le chlorure de méthylène, le chloroforme, le tétrachlorure de carbone, le trifluorométhylbenzène, le n-butanol, l'isobutanol, l'alcool amylique, l'alcool isoamylique, l'hexanol, le cyclohexanol, le 2-éthylhexanol, l'isooctanol, le sec-octanol, le butanone, le pentanone, l'hexanone, la cyclohexanone, la méthylisobutylcétone, ou un mélange de deux ou plus de ceux-ci.

9. Processus selon la revendication 1, dans lequel l'acide aminé à chaîne longue est l'acide 9-aminononanoïque, l'acide 11-aminoundécanoïque ou l'acide 13-aminotridécanoïque.

10. Processus selon la revendication 1, dans lequel l'acide aminé à chaîne longue est l'acide 11-aminoundécanoïque.

11. Processus selon la revendication 1, dans lequel l'acide dibasique à chaîne longue est l'acide sébacique, l'acide dodécanedioïque ou l'acide brassylique.

12. Processus selon la revendication 1, dans lequel l'acide dibasique à chaîne longue est l'acide dodécanedioïque.

13. Processus selon la revendication 1, dans lequel l'acide alcanoïque à chaîne courte est l'acide heptanoïque.

14. Processus selon la revendication 1, dans lequel l'alkylamine est la n-hexylamine.
